# FASCICULE DE BREVET EUROPEEN

(11) **EP 0 555 466 B1**
(45) Date de publication et mention de la délivrance du brevet: **30.10.1996**
(21) Numéro de dépôt: 92919419.9
(22) Date de dépôt: 01.09.1992
(51) Int. Cl.: A23L 1/23

(54) **PROCEDE D'OBTENTION D'AROME NATUREL DE VANILLE PAR TRAITEMENT ENZYMATIQUE DES GOUSSES DE VANILLE VERTE ET AROME OBTENU**
VERFAHREN ZUR HERSTELLUNG VON EINEM NATÜRLICHEN VANILLE-EXTRAKT DURCH ENZYMATISCHE VERARBEITUNG VON GRÜNEN VANILLEHÜLSEN UND SO ERHALTENER EXTRAKT
PROCESS FOR THE PRODUCTION OF NATURAL VANILLA EXTRACT BY ENZYMATIC PROCESSING OF GREEN VANILLA PODS, AND EXTRACT THEREBY OBTAINED

(30) Priorité: 03.09.1991 FR 9110873
(43) Date de publication de la demande: 18.08.1993
(73) Titulaire: PERNOD-RICARD, F-75008 Paris (FR)
(72) Inventeur: BRUNERIE, Pascal, Marc, F-94440 Santeny (FR)
(74) Mandataire: Warcoin, Jacques
(86) Numéro de dépôt international: FR9200837
(87) Numéro de publication internationale: WO9304597

(56) Documents cités:
- EP-A- 0 332 281
- EP-A- 0 416 713
- FR-A- 1 156 084
- FR-A- 2 443 265
- FR-A- 2 625 750
- FR-A- 2 634 979
- DATABASE WPIL Section Ch, Week 8316, Derwent Publications Ltd., London, GB; Class D, AN 83-38370K
- FOOD RESEARCH 1943, pages 343 - 351 F.E.ARANA 'Action of a beta-glucosidase in the curing of vanilla' cité dans la demande

## Description

La présente invention concerne un procédé d'obtention d'arôme naturel de vanille par traitement des gousses de vanille verte.

L'arôme de vanille comprend principalement comme constituants, la vanilline, l'acide vanillique, l'acide para-hydroxybenzoïque et l'aldéhyde para-hydroxybenzoïque. Ces constituants sont pratiquement absents de la gousse verte, même mature, et ne se forment que lentement dans la gousse après la cueillette.

Sur un plan industriel, les extraits de vanille (comprenant notamment les différents constituants énumérés ci-dessus) sont obtenus à partir de gousses ayant subit successivement de façon habituelle un échaudage, c'est-à-dire une immersion pendant 3 minutes dans l'eau à 65°C, un étuvage, pendant lequel les gousses perdent leur eau et prennent leur couleur brune, et une étape de maturation finale. Un tel traitement s'étale sur une période d'environ 9 mois pendant lesquels de nombreux contrôles de la qualité des gousses sont éffectués, ce qui entraîne une augmentation de son coût (Mémento de l'agronomie, 4è édition, Ministère de la coopération et du développement). Un tel procédé permet d'obtenir entre 20 et 30 g d'arôme de vanille par kilo de matière séche de gousses.

L'apparition de l'arôme de vanille au cours de ce traitement est dû en partie à l'hydrolyse d'un précurseur glucosilé, la glucovanilline qui se trouve dans la gousse verte à une teneur telle que, 50 g de vanilline pourrait être obtenu au cours de son hydrolyse lente lors de la maturation (Arana. F.E, 1943, Food Research, vol 8, pages 343-351). Une décomposition microbienne ou enzymatique de la vanilline est probablement responsable de la perte importante observée durant ce traitement.

Afin de pallier les inconvénients liés à ce procédé, il a déjà été proposé par le brevet FR-A-2 634 979 de congeler à une température comprise entre -5°C et -30°C les gousses vertes, puis de les réchauffer avant d'en extraire les constituants de l'arôme. Ce procédé permet de reccourcir la maturation.

On connaît également par le brevet français 1 156 084 un procédé de traitement de gousses de vanille en quatre étapes faisant intervenir dans l'étape I la découpe des gousses de vanille puis dans l'étape II l'hydratation et le pressage à travers un filtre de l'extrait obtenu à l'étape I.

L'étape III de concentration n'est effectuée que sur le filtrat (cf. page 2, colonne de droite avant dernier paragraphe) alors que le résidu solide est éliminé.

Dans l'étape IV avant ou pendant l'incubation, c'est-à-dire uniquement sur le filtrat, sont ajoutées une ou plusieurs enzymes pouvant modifier le goût, l'odeur, les caractéristique physiques et chimiques de l'extrait final vielli de vanille (page 3, colonne de gauche, 5ème paragraphe).

La demande de brevet japonais 58 043757 décrit un procédé de traitement de gousses de vanille par des microorganismes tels que levure ou bactérie.

On connaît par ailleurs par les demandes de brevet français 2 625 750 et 2 443 265 des procédés de production d'extraits de fruits ou de plantes par traitement enzymatique.

L'objet de la présente invention est de proposer un nouveau procédé qui permet de libérer la vanilline et les autres constituants aromatiques, contenus sous forme de glucosides dans la gousse verte.

Un autre objet de la présente invention est de proposer un procédé permettant d'obtenir un arôme de vanille avec un excellent rendement.

Selon la présente invention, le procédé d'obtention d'arôme naturel de vanille est caractérisé en ce que l'on traite un broyat de gousses de vanille vertes, au moyen d'un système enzymatique capable de détruire les systèmes membranaires de cellules végétales et d'hydrolyser les glucosides, constitué d'une ou plusieurs enzymes possédant une activité β-glucosidase. Ces enzymes présentant une activité β-glucosidase sont bien connues de l'homme du métier et de nombreux systèmes à base de celles-ci sont commercialement disponibles.

Le fait d'utiliser un broyat permet de faciliter l'attaque enzymatique des membranes des cellules. Il est donc clair que plus le broyage sera fin, plus l'attaque enzymatique en sera facilitée, compte tenu naturellement des contraintes liées à la mise en oeuvre industrielle d'un tel procédé. Par gousses vertes de vanille, on entend des gousses mures qui ont été fraîchement récoltées. En général, le procédé doit être mis en oeuvre quelques jours après la récolte, généralement entre 1 et 12 jours après celle-ci. L'utilisation du broyat brut évite par ailleurs toute étape d'extraction préalable.

Par système enzymatique capable de détruire les systèmes membranaires des cellules végétales, on entend tous les systèmes qui peuvent liquéfier les fruits, les légumes et en général toutes les cellules végétales pouvant servir d'aliment.

Avantageusement, on utilisera les systèmes enzymatiques particulièrement efficaces pour liquéfier les gousses de vanille verte.

De façon générale, on pourra utiliser une ou plusieurs préparations enzymatiques choisies parmi les pectinases, les cellulases, les hémicellulases ou les celliobiases, présentant une ou plusieurs activités β-glucosidases. Ces enzymes peuvent être utilisées seules ou en mélange.

D'une façon générale, il est bien entendu, que l'invention couvre tous les systèmes enzymatiques et notamment ceux ayant une activité glucosidase qui permettent de libérer l'arôme de vanille potentiellement contenu dans les gousses vertes de vanille. L'homme de métier sera à même par des manipulations simples (comme celles par exemple illustrées dans les exemples qui seront décrits à la suite de la présente description), de choisir les systèmes enzymatiques qui conviennent.

Selon une caractéristique avantageuse du procédé selon l'invention et afin de faciliter le broyage, les gousses vertes sont hydratées puis broyées. Généralement sans que cela soit une condition essentielle de la présente invention la quantité d'eau que l'on pourra ajouter, sera équivalente au poids de matériel végétal. Avantageusement, le broyat est centrifugé, filtré et redilué à l'alcool éthylique.

Le système enzymatique comprend avantageusement 10 à 1000 unités d'activité béta-glucosidase par gramme de gousses de vanille vertes, et de préférence de 20 à 500 unités d'activité enzymatiques béta-glucosidase. On a constaté qu'un intervalle compris entre 40 et 400 unités d'activité enzymatique était encore plus avantageux pour la mise en oeuvre du procédé selon l'invention.

L'incubation est réalisée à un pH qui est avantageusement compris entre 3 et 7, et de préférence 4 et 6 et encore plus avantageusement aux environs de 5. Le pH naturel du broyat obtenu étant égal à 5, ce broyat est donc naturellement à la valeur optimale.

De préférence également, le procédé est mis en oeuvre sous agitation pendant une durée suffisante pour permettre la libération de l'arôme de vanille. Avantageusement, cette durée sera supérieure à 2 heures à température ambiante. Cette température pourra être augmentée ou diminuée tout en prenant garde d'une part, à ne pas dépasser une température limite pouvant conduire à une dégradation de l'arôme de vanille, et d'autre part à ne pas abaisser de manière trop sensible la température risquant alors de bloquer les réactions souhaitées. Elles sera comprise généralement entre 10 et 60°C, de préférence entre 30-40°C

De façon avantageuse, la durée de l'incubation sera comprise entre 3 et 30 heures. On a constaté néanmoins, que la libération était généralement totale après quelques heures d'incubation, celle-ci pouvant être appréciée par l'homme de métier au moyen d'analyses en chromatographie liquide haute pression. Il est normalement conseillé de ne pas aller au delà de 24 heures.

Après incubation, la phase liquide contenant l'arôme de vanille, est séparée de la phase solide contenant notamment les résidus cellulaires insolubles. Cette séparation peut être effectuée par exemple, par filtration et/ou par centrifugation.

La phase liquide contenant l'arôme naturel de vanille peut ensuite être utilisée soit directement soit après concentration de l'extrait aromatique. Cette concentration peut être effectuée par évaporation, éventuellement sous vide puis filtration. Elle peut également être effectuée par extraction au solvant et évaporation subséquente de celui-ci. Les exemples ci-dessous illustrent l'invention sans toutefois la limiter :

Selon un mode opératoire général, les gousses vertes, après la récolte, sont broyées au mixeur après addition d'une quantité d'eau équivalente au poids de matériel végétal. L'incubation avec les enzymes est réalisée au pH naturel du broyat obtenu, c'est-à-dire à un pH de 5 environ sous agitation.

A la fin de la réaction, de l'alcool à 96 % est ajouté de façon à obtenir un milieu hydroalcoolique à 50 %. L'échantillon est ensuite filtré.

### EXEMPLE 1 : ESSAIS SUR DIFFERENTS SYSTEMES ENZYMATIQUES

Plusieurs types de préparations enzymatiques industrielles ont été testées de façon à en sélectionner une ou plusieurs possédant une activité glucosidase importante et dont la spécificité pouvait permettre de libérer la vanilline et les autres constituants volatils.

Les essais sont effectués avec les systèmes enzymatiques suivants (le symbole u est l'unité d'activité glucosidase) :

| | |
|---|---|
| A : sans système enzymatique (témoin) | |
| B : pectinase et bêta-glucosidase | 50 u/mg |
| C : pectinase/glucosidase et cellulase | 17 u/mg |
| D : pectinase/glucosidase | 79 u/mg |
| E : pectinase hemicellulase et glucosidase | 4455 u/mg |

Après incubation, le broyat est centrifugé. L'analyse et la détermination de la quantité de vanilline sont réalisées par chromatographie en phase liquide (HPLC) sur le surnageant.

Les résultats obtenus sont présentés dans le tableau ci-après :

| Type d'enzyme | Témoin sans enzyme | A | B | C | D | E |
|---|---|---|---|---|---|---|
| Quantité de Vanilline en g/kg de matière végétale | 0,355 | 0,319 | 1,3 | 3,19 | 1,5 | 6,5 |

L'enzyme E est une pectinase utilisée habituellement pour la liquéfaction des jus de fruit, celle-ci est la mieux adaptée à notre procédé. En effet, ses propriétés liquéfiantes permettent de détruire les systèmes membranaires de la cellule végétale libérant ainsi le contenu cytoplasmique permettant ainsi à l'activité glucosidase qu'elle contient de s'exprimer.

### EXEMPLE 2 : NOMBRE D'UNITES D'ENZYMES

On a déterminé la quantité de préparation enzymatique optimum pour la libération de l'arôme. Cette activité est déterminée à 30°C en milieu tamponné citrate phosphate pH = 5 à partir du p.nitrophenyl glucoside comme substrat, sur le système enzymatique E, une unité d'activité correspondant à l'hydrolyse d'une micromole de substrat par minute.

Les résultats sont indiqués dans le tableau ci-dessous :

| | | | | | |
|---|---|---|---|---|---|
| Quantité d'enzymes en unité d'activité /kg de gousses vertes x 10³ | 0 | 90 | 220 | 310 | 450 |
| Quantité de Vanilline en g/kg de gousses vertes obtenue après 20 h d'incubation | 1,5 | 2,9 | 3,5 | 3,6 | 3,5 |

Ce tableau montre que l'activité optimum se situe autour de 220 unités d'activité enzymatique pour 1 g de gousse verte mis en oeuvre.

### EXEMPLE 3 : DUREE D'INCUBATION

Différents prélèvements au cours du temps ont été effectués de façon à déterminer le temps d'incubation nécessaire à l'obtention d'une teneur maximale en vanilline libre dans le milieu. A 50 g de gousses vertes broyées dans 50 cc d'eau, sont ajoutées 12 x 10³ unités d'activité glucosidase (système E). Les résultats ci-dessous montrent que la quantité maximum de vanilline libre dans le milieu est atteinte après environ 7 heures d'incubation.

| Incubation à 30°C | | | | | | | |
|---|---|---|---|---|---|---|---|
| Temps d'incubation | TO | 1H | 2H | 4H | 8H | 10H | 25H |
| Quantité de vanilline en g/kg | 1,1 | 1,4 | 1,7 | 2,2 | 2,9 | 3,2 | 3,4 |

| Incubation à 37°C | | | | | | | |
|---|---|---|---|---|---|---|---|
| Temps d'incubation | TO | 1H | 2H | 4H | 6H | 25H | |
| Quantité de vanilline en g/kg | 1,1 | 2,3 | 2,9 | 3,6 | 3,9 | 4 | |

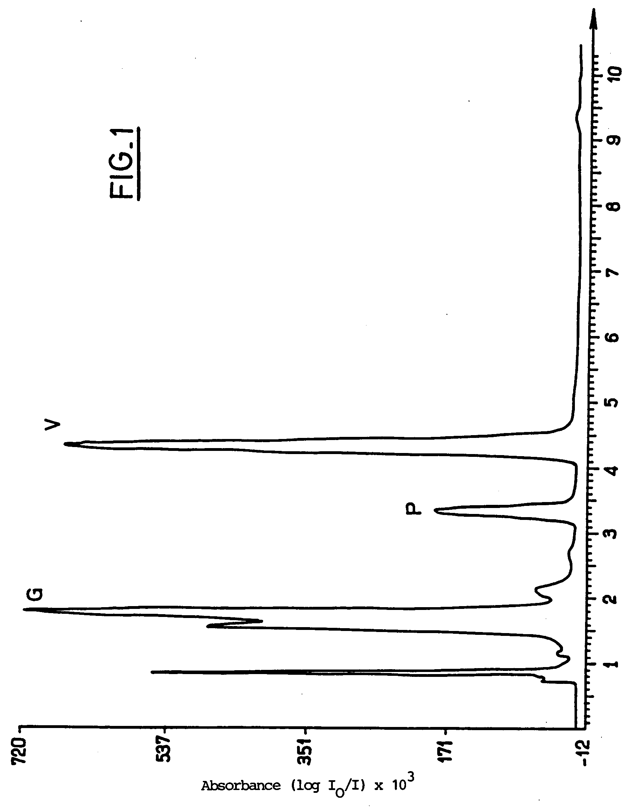

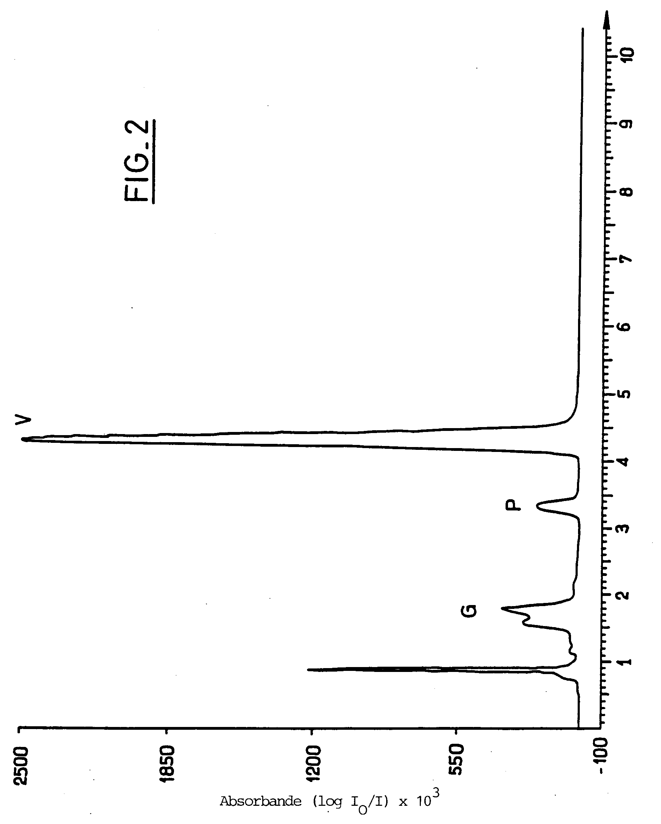

Les figures 1 et 2 annexées illustrent pour le dernier exemple, le chromatogramme obtenu après 20 heures sans système enzymatique (figure 1) et avec système enzymatique (figure 2).

Les méthodes d'analyse ont été les suivantes pour les exemples ci-dessus :

De l'alcool à 96 % est ajouté de façon à obtenir un milieu hydroalcooloqie à 50 %.

L'échantillon de milieu est filtré et injecté directement en HPLC.
- Appareil :: Hewlett Packard 1090, détecteur UV
Colonne Hewlett Packard ODS Hypersil
Solvant A : Tampon citrate phosphate pH : 4,66
Solvant B : acétonitrile
Débit : 0,3 ml/min.
Absorption à 300 nm
- Gradient :: T0 : 0 % de solvant B
10' : 15 % de solvant B
15' : 80 % de solvant B
20' : 10 % de solvant B

Les figures 1 et 2 annexées sont des chromatogrammes de broyat de gousses vertes.
- **Figure 1**:: Chromatogramme à 300 nm du broyat de gousses vertes, témoin sans enzymes.
G : Glucovanilline, V : Vanilline
P : Parahydroxy benzaldehyde.
- **Figure 2**:: Chromatogramme à 300 nm du broyat de gousses vertes, après 20 H d'incubation en présence d'enzyme :
G : Glucovanilline, V : Vanilline
P : parahydroxy benzaldehyde.

## Revendications

1. Procédé d'obtention d'arôme naturel de vanille, caractérisé en ce que l'on traite un broyat de gousses de vanille vertes au moyen d'un système enzymatique, capable de détruire les systèmes membranaires des cellules végétales et d'hydrolyser les glucosides, et comprenant d'une ou plusieurs enzymes possédant une activité bêta-glucosidase.

2. Procédé selon la revendication 1, caractérisé en ce que les systèmes enzymatiques sont choisis parmi les groupes suivants : les pectinases, les cellulases, les hémicellulases ou les exoglycosidases, comme les celliobiases.

3. Procédé selon l'une des revendications précédentes, caractérisé en ce que l'on utilise 20 à 500 unités d'activité enzymatique glucosidase par gramme de gousses de vanille vertes.

4. Procédé selon la revendication 3, caractérisé en ce que l'on utilise 40 à 400 unités d'activité enzymatiques par gramme de gousses de vanille vertes.

5. Procédé selon l'une des revendications précédentes, caractérisé en ce que le pH est compris entre 3 et 7, de préférence environ 5.

6. Procédé selon l'une des revendications précédentes, caractérisé en ce que la durée d'incubation du mélange de broyat et du système enzymatique est supérieure à deux heures, de préférence entre 3 et 30 heures.

7. Procédé selon l'une des revendications précédentes, caractérisé en ce que la température est comprise entre 10 et 60°C, de préférence 30 et 40°C.

8. Procédé selon l'une des revendications précédentes, caractérisé en ce que les gousses vertes sont hydratées avant broyage.

9. Procédé selon l'une des revendications précédentes, caractérisé en ce que après incubation la phase liquide est séparée de la phase solide, la phase liquide étant ensuite récupérée, l'extrait aromatique étant éventuellement isolé.

## Claims

1. Process for the production of natural vanilla flavour, characterized in that a ground product of green vanilla pods is treated by means of an enzymatic system capable of destroying the membrane systems of plant cells and of hydrolysing the glucosides, and comprising one or more enzymes which possess a beta-glucosidase activity.

2. Process according to Claim 2, characterized in that the enzymatic systems are chosen from the following groups: pectinases, cellulases, hemicellulases or exoglycosidases, such as celliobiases.

3. Process according to one of the preceding claims, characterized in that 20 to 500 units of glucosidase enzymatic activity per gram of green vanilla pods are used.

4. Process according to Claim 3, characterized in that 40 to 400 units of enzymatic activity per gram of green vanilla pods are used.

5. Process according to one of the preceding claims, characterized in that the pH is between 3 and 7, preferably about 5.

6. Process according to one of the preceding claims, characterized in that the period of incubation of the mixture of ground product and the enzymatic system is greater than two hours, preferably between 3 and 30 hours.

7. Process according to one of the preceding claims, characterized in that the temperature is between 10 and 60°C, preferably 30 and 40°C.

8. Process according to one of the preceding claims, characterized in that the green pods are hydrated before grinding.

9. Process according to one of the preceding claims, characterized in that, after incubation, the liquid phase is separated from the solid phase, the liquid phase then being recovered, the flavour extract being optionally isolated.

## Patentansprüche

1. Verfahren zur Gewinnung von natürlichem Vanille-Aroma, dadurch gekennzeichnet, daß man ein Mahlgut von grünen Vanilleschoten mit einem enzymatischen System behandelt, das die Membransysteme der Pflanzenzellen zerstören und die Glucoside hydrolysieren kann und ein oder mehrere Enzyme umfaßt, die eine β-Glucosidase-Aktivität aufweisen.

2. Verfahren nach Anspruch 1, dadurch gekennzeichnet, daß die enzymatischen Systeme ausgewählt werden aus der Gruppe der Pectinasen, der Cellulasen, der Hemicellulasen oder der Exoglycosidasen wie der Cellobiasen.

3. Verfahren nach einem der vorhergehenden Ansprüche, dadurch gekennzeichnet, daß man 20 bis 500 enzymatische Glucosidase-Aktivitäts-Einheiten pro Gramm grüne Vanilleschoten verwendet.

4. Verfahren nach Anspruch 3, dadurch gekennzeichnet, daß man 40 bis 400 enzymatische Aktivitäts-Einheiten pro Gramm grüne Vanilleschoten verwendet.

5. Verfahren nach einem der vorhergehenden Ansprüche, dadurch gekennzeichnet, daß der pH-Wert zwischen 3 und 7, vorzugsweise bei etwa 5, liegt.

6. Verfahren nach einem der vorhergehenden Ansprüche, dadurch gekennzeichnet, daß die Dauer der Inkubation der Mischung aus dem Mahlgut und dem enzymatischen System mehr als 2 h, vorzugsweise 3 bis 30 h, beträgt.

7. Verfahren nach einem der vorhergehenden Ansprüche, dadurch gekennzeichnet, daß die Temperatur zwischen 10 und 60°C, vorzugsweise zwischen 30 und 40°C, liegt.

8. Verfahren nach einem der vorhergehenden Ansprüche, dadurch gekennzeichnet, daß die grünen Schoten vor dem Mahlen (Zerkleinern) hydratisiert werden.

9. Verfahren nach einem der vorhergehenden Ansprüche, dadurch gekennzeichnet, daß nach der Inkubation die flüssige Phase von der festen Phase getrennt wird, wobei die flüssige Phase anschließend Zurückgewonnen und der aromatische Extrakt gegebenenfalls isoliert werden.
